Europäisches Patentamt

European Patent Office

Office européen des brevets

(11) Veröffentlichungsnummer: **0 361 134**
**A2**

## (12) EUROPÄISCHE PATENTANMELDUNG

(21) Anmeldenummer: 89116291.9

(22) Anmeldetag: 04.09.89

(51) Int. Cl.⁵: **A61M 16/01**

(30) Priorität: 27.09.88 CH 3570/88

(43) Veröffentlichungstag der Anmeldung:
**04.04.90 Patentblatt 90/14**

(84) Benannte Vertragsstaaten:
**DE FR GB**

(71) Anmelder: **TECAN MEDICAL AG**
**Rossbodenstrasse 15**
**CH-7000 Chur(CH)**

(72) Erfinder: **Jösler, Hans-Jürg**
**Via Salens 34**
**CH-7402 Bonaduz(CH)**
Erfinder: **Morscher, Elmar**
**Via Calundis 30**
**CH-7013 Domat/Ems(CH)**
Erfinder: **Neher, Thomas**
**Rietberg**
**CH-7415 Pratval(CH)**
Erfinder: **Zogg, Jean-Marie**
**Via Salens 30**
**CH-7402 Bonaduz(CH)**

(74) Vertreter: **Lusuardi, Werther Giovanni et al**
**Dr. Lusuardi AG Kreuzbühlstrasse 8**
**CH-8008 Zürich(CH)**

(54) Zusammengefasste, austauschbare Frischgasabgabeeinheit für Anästhesiegeräte.

㊳ Bei dieser zusammengefassten, austauschbaren Frischgasabgabeeinheit für Anästhesiegeräte werden

a) die Zuführung der Anästhesiegase,

b) das Dosieren und Mischen der Anästhesiegase,

c) die Dosierung und Beimischung des Anästhetikums zum Gasgemisch, und

d) die Einführung des mit Anästhetikum angereicherten Gasgemisches in den Patientenkreislauf von einer zentralen Steuereinheit (20) geregelt und überwacht. Damit kann eine wesentlich verbesserte Sicherheit und Genauigkeit im Betrieb von Anästhesiegeräten erreicht werden.

Fig 1

## Zusammengefasste, austauschbare Frischgasabgabeeinheit für Anästhesiegeräte

Die Erfindung bezieht sich auf eine zusammengefasste, austauschbare Frischgasabgabeeinheit für Anästhesiegeräte.

Anästhesiegeräte beeinhalten im wesentlichen drei verschiedene Funktionen:

    1. Dosierung der Narkosemittel (Frischgasabgabeeinheit),

    2. Aufrechterhaltung der Beatmung (Beatmungsgerät) und die

    3. Pneumatik (Schlauchsystem ev. mit Absorber).

Frischgasabgabeeinheiten für Anästhesiegeräte bestehen im wesentlichen aus vier Einzelkomponenten, nämlich:

    a) einer Anästhesiegasquelle,

    b) mindestens zwei Durchflussreglern (Rotameter) für den gesamten Gasfluss und die Einstellung der Konzentration,

    c) einem Verdampfer für das Anästhetikum, und

    d) einem Frischgasauslass.

Bei den bekannten Frischgasabgabeeinheiten sind diese und allfällige weitere nicht essentielle Komponenten (z.B. Verdampferblockierung, Sauerstoffspülung, Sauerstoffalarm, Lachgasabschaltung, Druckregler, Einwegventile u.ä.) lediglich mechanisch und pneumatisch miteinander verbunden, so dass mit zunehmender Anzahl von Einzelkomponenten auch die Gefahr von Verwechslungen und Undichtigkeiten sprunghaft zunimmt.

Ein weiterer Nachteil der Frischgasabgabeeinheiten gemäss dem Stand der Technik liegt darin begründet, dass die Sauerstoffkonzentration und der gesamte Gasfluss nicht unabhängig voneinander an die jeweiligen Erfordernisse geändert werden können, so dass der Anästhesist die Sauerstoffkonzentration nach jeder Neueinstellung selbst berechnen und mittels eines iterativen Verfahren einem gewünschten Wert annähern muss. Abgesehen davon, dass die in konventionellen Frischgasabgabeeinheiten zur Dosierung der Anästhesiegase verwendeten Rotameter - vor allem im unteren Messbereich - sehr ungenau sind, kann es wegen der fehlenden internationalen Normierung immer wieder zu Verwechslungen mit fatalem Resultat kommen. Auch die als Einzelkomponenten verwendeten Verdampfer können mit dem falschen Anästhetikum aufgefüllt werden, was ebenfalls tödliche Folgen haben kann. Aber auch das in den nach dem Prinzip eines Verdunsters arbeitenden Verdampfern als Stabilisator für das Anästhetikum verwendete Thymol kann zu Verstopfungen und damit zu erheblichen Fehlern führen. Überhaupt ist die Genauigkeit der konventionellen Verdampfer sehr zweifelhaft, da die Abgabe des Anästhetikums von vielen unbeeinflussbaren Faktoren abhängt (Umgebungstemperatur, Umgebungsdruck, Konzentration der als Träger fungierenden Anästhesiegase, Löslichkeit des Lachgases im Anästhetikum).

Hier will die Erfindung Abhilfe schaffen. Der Erfindung liegt die Aufgabe zugrunde, eine als Einheit funktionierende Frischgasabgabeeinheit für Anästhesiegeräte zu schaffen, die einerseits zusammengefasst aufgebaut und somit für beliebige Anästhesiegeräte austauschbar ist, und bei der anderseits sämtliche Funktionen zentral gesteuert und überwacht werden können.

Eine weitere Aufgabe liegt in der verbesserten Beimischung des Anästhetikums zum Gasgemisch.

Die Erfindung löst die gestellte Aufgabe mit einer zusammengefassten, austauschbaren Frischgasabgabeeinheit, welche die Merkmale des Anspruchs 1 aufweist.

Die durch die Erfindung erreichten Vorteile sind im wesentlichen darin zu sehen, dass dank der erfindungsgemässen Vorrichtung eine wesentlich verbesserte Sicherheit und Genauigkeit im Betrieb von Anästhesiegeräten erreicht werden kann.

Insbesondere ergeben sich folgende Vorteile:

- Die Sauerstoffkonzentration und der gesamte Gasfluss können dank der zentralen Steuerung mittels zweier voneinander unabhängiger Regler eingestellt werden. Das mühsame iterative Verfahren entfällt.

- Eine hohe Genauigkeit von ± 2 Vol% Sauerstoff ist über den gesamten Durchflussbereich (0,2 bis 15 l/min) erreichbar.

- Die Sauerstoffkonzentration bleibt auch bei einem Verdampferwechsel konstant.

- Das Auftreten einer hypoxischen Gasmischung wird automatisch verhindert.

- Im Falle eines Sauerstoffdruckabfalls wird automatisch auf Luftbetrieb umgeschaltet.

- Bei gefährlichen Alarmzuständen wird automatisch eine Sauerstoffspülung (oxygen flush) ausgelöst.

- Die Sauerstoffkonzentration ist von den Umgebungsparametern unabhängig.

- Der Gehalt an Anästhetikum kann über den gesamten Durchflussbereich (0,2 bis 15 l/min) äusserst genau dosiert werden.

- Alle Parameter, welche die Konzentrationen von Sauerstoff und Anästhetikum beeinflussen, können dank der zentralen Steuereinheit automatisch kompensiert werden.

- Dank der erfindungsgemässen zentralen Steuerung und Überwachung kann für jeden einzelnen Patienten der exakte Verbrauch an teurem Anästhetikum berechnet werden, so dass eine aufwandgerechte Spesenabrechnung durch die Spitalverwaltung möglich wird.

- Die erfindungsgemässe Vorrichtung erlaubt erstmals die automatische Narkoseeinleitung (hoher Anfangsfluss mit nachfolgender automatischer Reduktion des Flusses bis zur für die Aufrechterhaltung der Narkose notwendigen Minimalmenge).

- Die Vorratsanzeige an Anästhetikum kann nun automatisch erfasst werden und bei Unterschreitung eines gewissen Vorratswertes zur Auslösung eines Alarms verwendet werden.

- Die Auffüllung des Verdampfers erübrigt sich.

- Im Falle von Druckabfällen im System erfolgt eine automatische Alarmierung.

- Es können Gasquellen unterschiedlichen Druckes (2 - 6 bar) verwendet werden.

- Dank des zentralen Rechners kann eine Rückkoppelung der Ausgangswerte zu den Eingangswerten erfolgen.

- Bestehende Anästhesiegeräte können auf einfache Weise mit der erfindungsgemässen Vorrichtung umgerüstet werden.

- Dank der getakteten Zugabe von Sauerstoff und Lachgas mit einem einzigen Durchflussmesser bleibt die hohe Messgenauigkeit auch bei einem allfälligen Fehler des Durchflussmessers erhalten, da sich der Fehler auskompensiert.

- Dank der Abgabe einer genau vorbestimmten Menge Anästhetikum sind die Konzentrationsfehler minimal.

Ein Ausführungsbeispiel der Erfindung, welches zugleich das Funktionsprinzip erläutert, ist in der Zeichnung dargestellt und wird im folgenden näher beschrieben.

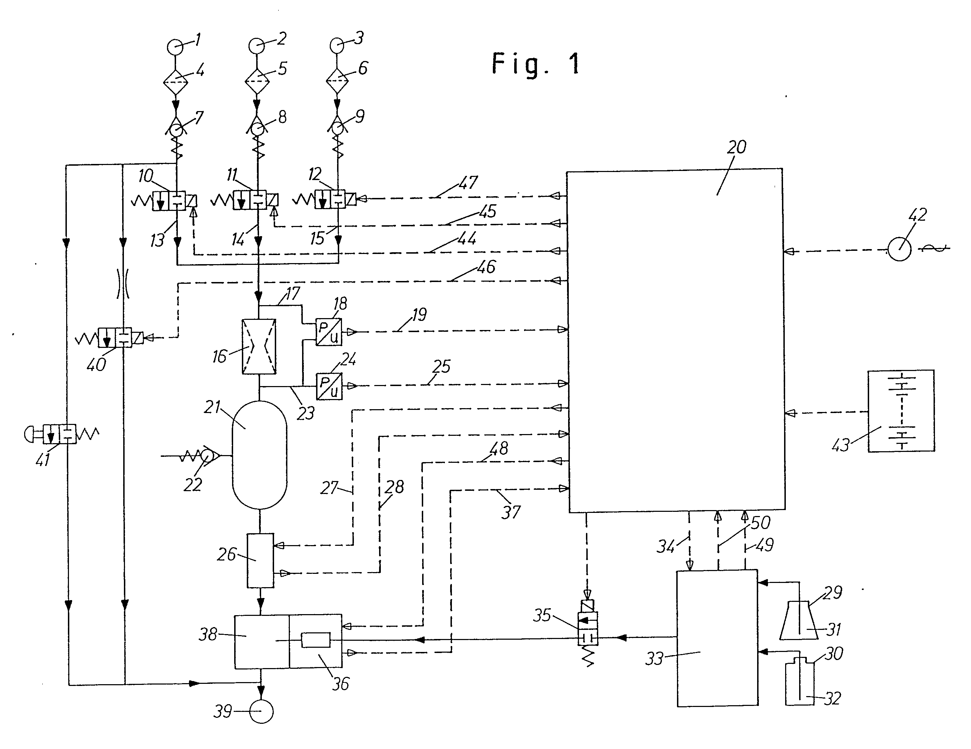

Figur 1 stellt ein kombiniertes elektrisches und pneumatisches Schaltschema dar.

Anhand der Fig. 1 werden vorerst die einzelnen Elemente der erfindungsgemässen Vorrichtung beschrieben.

Die Gaseingänge 1,2,3 sind die Schnittstellen zwischen dem Anästhesiegerät und der Gasversorgungsanlage oder den Gasflaschen des Spitals. Für die Anästhesie werden normalerweise Sauerstoff 1 und Lachgas 2 verwendet. Es können aber weitere Gaseingänge, wie z.B. für Druckluft 3, Kohlendioxid, Helium u.s.w. verwendet werden. Der Eingangsdruck liegt typischerweise bei 3,5 bar.

Den Gaseingängen 1,2,3 nachgeschaltet sind Filter 4,5,6, welche den Durchfluss von fremden, insbesondere von festen Partikeln, die aus den Gaseingängen 1,2,3 stammen könnten, verhindern sollen. Damit wird gewährleistet, dass nur saubere Gase zum Patienten gelangen.

Den Filtern 4,5,6 nachgeschaltet sind Einwegventile 7,8,9, welche gewährleisten sollen, dass der Gasfluss nur in einer Richtung stattfinden kann. Auf diese Weise wird jede Rückmischung der Gase verhindert.

Den Einwegventilen 7,8,9 nachgeschaltet sind Gasventile 10,11,12 (Sauerstoffventil 10, Lachgasventil 11 und Druckluftventil 12), welche elektrisch betrieben sind. Das Sauerstoffventil ist über die Sauerstoffventilsteuerleitung 44, das Lachgasventil 11 über die Lachgasventilsteuerleitung 45 und das Druckluftventil 12 über die Druckluftventilsteuerleitung 47 mit der elektronischen Steuereinheit 20 verbunden. Liegt eine elektrische Spannung an, so schaltet das entsprechende Ventil durch und ein Gasstrom kann durchfliessen.

Die Gasleitungen 13,14,15 welche von den Gaseingängen 1,2,3 zu den Filtern 4,5,6 durch die Einwegventile 7,8,9 und Gasventile 10,11,12 führen, vereinigen sich unterhalb der Gasventile 10,11,12 und münden in eine Messblende 16. Die Messblende 16 ist eine Laminarblende, bei der ein proportional zum Gasdurchfluss stehendender Druckabfall auftritt. Von der Messblende 16 führen die Leitungen 17 und 23 zum Durchfluss druckumsetzer 18, der den mittels der Messblende 16 gemessenen, differentiellen Druckabfall in ein elektrisches Signal 19 umwandelt, welches proportional zum Durchfluss ist und das der elektronischen Steuereinheit 20 zugeführt wird. Die durch die Laminarblende 16 strömenden Gase werden einem Vorratsbehälter 21 mit einem vorgegebenen Volumen zugeführt, welcher die einströmenden Gase für eine bestimmte Zeit speichert und deren Durchmischung gestattet. Die vermischten Gase im Vorratsbehälter 21 befinden sich typischerweise unter einem Druck von 200 - 1000 mbar.

Für den Fall, dass eines der Gasventile 10,11,12 ausfallen könnte, limitiert ein Überdruckventil 22 den maximalen Druck im Vorratsbehälter 21 auf einen vorgegebenen Wert. Die Einstellung beträgt typischerweise 1,2 bar.

Ein über die Leitung 23 mit dem Vorratsbehälter 21 verbundener Behälterdruckmessaufnehmer 24 wandelt den gemessenen Druck im Vorratsbehälter 21 in ein elektrisches Signal 25 um, das der elektronischen Steuereinheit 20 zugeführt wird.

Ein dem Vorratsbehälter 21 nachgeschaltetes Flussregelventil 26 (z.B. ein Servoventil oder Massflowcontroller) erzeugt einen kontinuierlichen Gasfluss aus dem Vorratsbehälter 21 heraus, welcher in Funktion zum elektrischen Sollwert 27 für den Fluss steht. Der tatsächliche Fluss wird durch die Flussmessignalleitung 28 erfasst, welche zur elektronischen Steuereinheit 20 führt.

Anästhetikabehälter 29, 30 oder weitere in der Form von Flaschen oder Recipients beinhalten das in flüssigem Zustand vorliegende, volatile Anästhetikum 31,32. Wenn verschiedene Anästhetika 31,32 gebraucht werden, können ein oder mehrere Anästhetikabehälter 29,30 angeschlossen werden.

Eine Flüssigkeitsdosiereinrichtung 33 (z.B. eine gesteuerte Spritze oder Pumpe) entnimmt aus dem Anästhetikabehälter 29,30 eine vorgegebene Menge flüssiges Anästhetikum 31,32 und befördert sie weiter zu einem Sicherheitsventil 35. Die zu dosierende Menge wird über die Flüssigkeits-Sollwert-Leitung 34 vorgegeben. Das Sicherheitsventil 35 verhindert, dass im ausgeschalteten Zustand oder bei einer Betriebsstörung der Frischgasabgabeeinheit Flüssigkeit 31,32 weiterbefördert wird. Eine Verdampfungseinheit 36 wandelt die dosierte Flüssigkeit 31,32 mittels über die Leitung 48 zugeführte elektrische Energie in den dampfförmigen Zustand um. Die dazu notwendige Energie ist ein Mass für die Menge der verdampften Flüssigkeit. Die Temperatur der Verdampfungseinheit 36 wird konstant gehalten und über das Temperaturmessignal 37 erfasst und der elektronischen Steuereinheit 20 zugeführt.

In einer Mischkammer 38 geschieht das Durchmischen des von der Verdampfungseinheit 36 gelieferten dampfförmigen Anästhetikums mit dem durch das Flussregelventil 26 geregelten Gasfluss aus dem Vorratsbehälter 21. Das in der Mischkammer 38 zubereitete Frischgas gelangt durch einen Ausgang 39 zum (nicht dargestellten) Patientenschlauchsystem.

Im Falle einer Betriebsstörung gewährleistet ein Notfallventil 40 automatisch einen Sauerstofffluss zum Ausgang 39 und damit zum Patienten. Dieses Notfallventil 40 wird über die Leitung Notfallventilsteuerung 46 von der elektronischen Steuereinheit 20 aktiviert.

Über ein Flushventil 41 hat der Anwender die Möglichkeit einen vorgegebenen hohen Sauerstoffgasfluss (typischerweise 40 Liter/min) zum Patientenschlauchsystem und damit zum Patienten fliessen zu lassen. Dieses Flushventil 41 wird direkt vom Anwender betätigt.

Die elektronische Steuereinheit 20 regelt und steuert den gesamten Ablauf der Gasdurchmischung, der Gasflussdosierung und der Anästhetikum-Beimischung. Anhand der vom Anwender gewünschten Parameter, wie z.B. die gewünschte Gaskonzentration, der gewünschte Fluss und die gewünschte Anästhetikumkonzentration, werden die entsprechenden Baugruppen, Einheiten und Komponenten der erfindungsgemässen Vorrichtung gesteuert und/oder geregelt.

Eine Netzstromversorgung 42 hat die Aufgabe die Elektronik und die entsprechenden Baugruppen mit elektrischer Energie zu versorgen. Eine Batteriestromversorgung 43 gewährleistet, dass das ganze Gerät bei einem Stromausfall oder bei einer fehlenden Netzstromversorgung 42 für eine bestimmte Zeit funktionsfähig bleibt.

Es folgt nun eine detaillierte Beschreibung der verschiedenen Funktionen der erfindungsgemässen Vorrichtung:

Gasmischung

Das Gasgemisch (z.B. Sauerstoff Lachgas; Sauerstoff Luft oder weitere Kombinationen) befindet sich im Vorratsbehälter 21 unter einem bestimmten maximalen Druck von beispielsweise 1000 mbar. Das Flussregelventil 26 entnimmt aus dem Vorratsbehälter 21 kontinuierlich einen konstanten Gasfluss von beispielsweise 1 Liter Minute. Dadurch sinkt der Druck im Vorratsbehälter 21. Sobald der Druck einen Mindestwert von beispielsweise 200 mbar erreicht, wird dies von der elektronischen Steuereinheit 20 über den Behälterdruckmessaufnehmer 24 und der Leitung Behälterdruckmessignal 25 detektiert. Nach Detektion der Mindestdruckgrenze wird von der elektronischen Steuereinheit 20 der Ladevorgang des Vorratsbehälters 21 gestartet.

Die elektronische Steuereinheit 20 berechnet nun anhand des vorhandenen Mindestdruckes, des gewünschten maximalen Behälterdrucks, des Abflusses über das Flussregelventil 26 und der geometrischen Abmessungen des Vorratsbehälters 21, das notwendige gesamte Volumen, welches wieder in den Vorratsbehälter 21 einfliessen muss um den gewünschten maximalen Behälterdruck wieder zu erreichen.

Anhand des errechneten Gesamtvolumens (z.B. 1 Liter) errechnet die elektronische Steuereinheit 20 aus der gewünschten Gaskonzentration von beispielsweise 35 Vol% Sauerstoff die Teilvolumina für Sauerstoff und Lachgas ( bei einer Sauerstoff/Lachgas-Mischung). Das Teilvolumen für den Sauerstoff ergibt sich aus der gewünschten Konzentration multipliziert mit dem errechneten Gesamtvolumen (z.B. 0,35 x 1 Liter = 350 ml). Das Teilvolumen für das Lachgas ergibt sich einfach aus dem verbleibenden Volumen um das Gesamtvolumen zu erreichen (z.B. 1 Liter - 350 ml = 650 ml).

Die elektronische Steuereinheit 20 schaltet nun das Sauerstoffventil 10 über die Sauerstoffventilsteuerleitung 44 solange durch, bis das Integral über die Zeit des Durchflussmessignals 19 (gemessenes abgegebenes Sauerstoffvolumen) den Wert des errechneten Teilvolumens für den Sauerstoff ergibt. Nachdem das Sauerstoffteilvolumen erreicht ist, schaltet die elektronische Steuereinheit 20 das Sauerstoffventil 10 ab. Der Sauerstoffgasfluss zum Vorratsbehälter 21 wird somit unterbrochen. Die elektronische Steuereinheit 20 schaltet nun das Lachgasventil 11 über die Lachgassteuerleitung 45 durch, solange bis das Integral über die Zeit des Durchflussmessignales 19 (gemessenes abgegebenes Lachgasvolumen) den Wert des errechneten Teilvolumens für das Lachgas ergibt. Beim Erreichen des Lachgasteilvolumens schaltet die elektronische Steuereinheit 20 das Lachgasventil 11 ab. Der Lachgasfluss zum Vorratsbehälter 21 wird damit unterbrochen und im Vorratsbehälter 21 herrscht wieder der gewünschte maximale Behälterdruck (z.B. 1000 mbar).

Der Vorratsbehälterdruck beginnt nun wieder zu sinken und sobald der Mindestdruck erreicht ist, wird der Ladevorgang durch die elektronische Steuereinheit 20 von neuem gestartet. Dank der Verwendung der gleichen Messblende 16 zum Ermitteln der Teilvolumina von Sauerstoff und Lachgas wird eine hohe Konzentrationsgenauigkeit erreicht, weil allfällige Ungenauigkeiten der Messblende 16 und des Durchflussdruckumsetzers 18 sich gegenseitig auskompensieren.

## Gasflussregelung

Die elektronische Steuereinheit 20 gibt an das Flussregelventil 26 über die Leitung Flussollwert 27 ein entsprechendes elektrisches Signal ab. Die Grösse des Signals ist abhängig vom eingestellten Gesamtfluss (z.B. 1 Liter/min). Das Flussregelventil 26 wird nun solange nachgestellt bis das über Leitung 28 übertragene Flussmessignal dem über Leitung 27 übertragenen Flussollwertsignal entspricht.

## Dosierung der volatilen Anästhetika

Anhand der vom Anwender gewünschten Anästhetikakonzentration gibt die elektronische Steuereinheit 20 über die Leitung Flüssigkeitssollwert 34 der Flüssigkeitsdosiereinheit 33 einen bestimmten Sollwert vor. Die Flüssigkeitsdosiereinheit 33 entnimmt aus dem Anästhetikabehälter 29,30 die notwendige Menge Anästhetikum 31 oder 32, meldet die entnommene Menge über die Leitung Flüssigkeitsistwert 50 an die elektronische Steuereinheit 20 und fördert diese Menge über ein Sicherheitsventil 35 zur Verdampfungseinheit 36. In der Verdampfungseinheit 36 wird, mittels in Wärme umgesetzte elektrische Energie, die Flüssigkeit verdampft. Die Energie, welche benötigt wird um die Temperatur der Verdampfungseinheit 36 konstant zu halten, ist abhängig von der zugeführten Flüssigkeitsmenge. Dadurch kann die verdampfte Flüssigkeitsmenge überwacht werden. Bei einer Betriebsstörung schaltet die elektronische Steuereinheit 20 das Sicherheitsventil 35 ab und die Flüssigkeitszufuhr zur Verdampfungseinheit 36 wird augenblicklich unterbrochen.

Der Anwender hat die Möglichkeit verschiedene Anästhetika 31,32 oder weitere einzusetzen. Entweder wählt er über die elektronische Steuereinheit 20 das gewünschte Anästhetikum 31,32 an und die Flüssigkeitsdosiereinheit 33 entnimmt dann dem entsprechenden Behälter 29 oder 30 die notwendige Flüssigkeitsmenge oder er kann durch gezieltes Anstecken des Anästhetikumbehälters 29 oder 30 das gewünschte Anästhetikum 31 oder 32 vorgeben. In letzterem Falle detektiert die Flüssigkeitsdosiereinheit 33, welches Anästhetikum 31 oder 32 im Gebrauch ist und gibt diese Information über die Leitung 49 an die elektronische Steuereinheit 20 weiter.

## Steuerung

Die elektronische Steuereinheit 20 besteht entweder aus einem oder mehreren in der Frischgasabgabeeinheit integrierten Mikrokontroller konventioneller Bauweise oder einem separaten Rechner, beispielsweise einem Personal Computer.

Die Eingangsgrössen können entweder direkt vom Anwender über geeignete Potentiometer, Tasten oder über eine genormte Schnittstelle von einem anderen Gerät, beispielsweise einem Computer, eingespiesen werden.

## Ansprüche

1. Zusammengefasste, austauschbare Frischgasabgabeeinheit für Anästhesiegeräte, dadurch gekennzeichnet, dass

a) die Zuführung der Anästhesiegase,

b) das Dosieren und Mischen der Anästhesiegase,

c) die Dosierung und Beimischung des Anästhetikums zum Gasgemisch, und

d) die Einführung des mit Anästhetikum angereicherten Gasgemisches in den Patientenkreislauf

von einer zentralen Steuereinheit (20) geregelt und überwacht ist.

2. Frischgasabgabeeinheit nach Anspruch 1, dadurch gekennzeichnet, dass das Mischen der Anästhesiegase mittels eines einzigen Durchflussmessers (16) erfolgt, wobei abwechslungsweise die durchströmenden Volumina der einzelnen Anästhesiegase gemessen werden und einem gemeinsamen Vorratsbehälter (21) zugeführt und dort miteinander vermischt werden.

3. Frischgasabgabeeinheit nach Anspruch 2, dadurch gekennzeichnet, dass der einzige Durchflussmesser (16) eine Laminarblende ist, bei welcher der Druckabfall proportional zum Fluss ist.

4. Frischgasabgabeeinheit nach einem der Ansprüche 1 bis 3, dadurch gekennzeichnet, dass das Dosieren des Frischgasflusses mittels eines einzigen Flussregelventils (26) erfolgt, wobei der Frischgasfluss geregelt wird.

5. Frischgasabgabeeinheit nach einem der Ansprüche 1 bis 4, dadurch gekennzeichnet, dass die Konzentration an Anästhetikum (31;32) unter Konstanthaltung der Sauerstoffkonzentration variierbar

ıst.

6. Frischgasabgabeeinheit nach einem der Ansprüche 1 bis 5, dadurch gekennzeichnet, dass eine wählbare Menge an flüssigem Anästhetikum (31;32) unter kontrollierten Bedingungen verdampft wird und gesamthaft dem Gasgemisch beigemischt wird.

7. Frischgasabgabeeinheit nach einem der Ansprüche 1 bis 6, dadurch gekennzeichnet, dass die Zusammensetzung des mit Anästhetikum (31;32) angereicherten Gasgemisches kontinuierlich überwacht wird und insbesondere die Konzentration an Sauerstoff und Anästhetikum ermittelt und angezeigt werden.

8. Frischgasabgabeeinheit nach einem der Ansprüche 1 bis 7, dadurch gekennzeichnet, dass die ermittelten Konzentrationswerte für Sauerstoff und Anästhetikum (31;32) zur Regelung der Eingangs-Dosierung dieser beiden Stoffe verwendet werden.

9. Frischgasabgabeeinheit nach einem der Ansprüche 1 bis 8, dadurch gekennzeichnet, dass die elektronische Steuereinheit (20) ausentweder aus einem oder mehreren in der Frischgasabgabeeinheit integrierten Mikrokontroller besteht.

Fig. 1